# EUROPEAN PATENT APPLICATION

(11) **EP 4 678 169 A1**
(43) Date of publication of application: **14.01.2026**
(21) Application number: 24187090.6
(22) Date of filing: 08.07.2024
(51) Int. Cl.: A61K 31/05, A61K 9/00, A61P 31/14

(54) **COMPOUNDS FOR USE IN THE TREATMENT AND PREVENTION OF INFECTIONS WITH RSV**

(71) Applicant: Szekeres, Thomas, 1010 Wien (AT); Jäger, Walter, 3021 Pressbaum (AT); Bánki, Zoltán, 6020 Innsbruck (AT)
(72) Inventor: Szekeres, Thomas, 1010 Wien (AT); Jäger, Walter, 3021 Pressbaum (AT); Bánki, Zoltán, 6020 Innsbruck (AT)
(74) Representative: SONN Patentanwälte GmbH & Co KG

(57) **Abstract**

The invention discloses 3,3',4,4',5,5'-hexahydroxy-trans-stilbene or a pharmaceutically acceptable ester thereof, for use in the treatment and prevention of infections with RSV in a human subject, especially for inhibiting human RSV.

## Description

The present invention relates to the treatment and prevention of diseases caused by respiratory syncytial virus (RSV).

RSV is a common respiratory virus that usually causes mild, cold-like symptoms. Most people recover in a week or two, but infants and older adults are more likely to develop severe RSV and need hospitalization.

RSV is a negative-sense, single-stranded RNA virus. The scientific name for this viral species is human orthopneumovirus. This is synonymous with human respiratory syncytial virus (hRSV), which is often shortened to just RSV. Accordingly, human RSV will be addressed herein as RSV, i.e. RSV and human RSV (hRSV) are used synonymously herein. It belongs to the genus Orthopneumovirus, family Pneumoviridae, order Mononegavirales. Its name comes from the fact that F proteins on the surface of the virus cause neighbouring cell membranes to merge, creating large multinucleated syncytia.

In May 2023, the US Food and Drug Administration (FDA) approved the first RSV vaccines, Arexvy (developed by GSK) and Abrysvo (developed by Pfizer). The prophylactic use of palivizumab or nirsevimab (both are monoclonal antibody treatments) can prevent RSV infection in high-risk infants.

Treatment for severe illness is primarily supportive, including oxygen therapy and more advanced breathing support with continuous positive airway pressure (CPAP) or nasal high flow oxygen, as required. In cases of severe respiratory failure, intubation and mechanical ventilation may be required. Ribavirin is an anti-viral medication licensed for the treatment of RSV in children. As already mentioned, RSV infection is usually not serious, but it can be a significant cause of morbidity and mortality in infants and in adults, particularly the elderly and those with underlying heart or lung diseases.

Accordingly, there is still a significant need to provide specific antiviral agents against RSV to combat the disease in human patients.

Thus, it is desirable to have additional methods for inhibiting the formation of RSV and/or for preventing and treating RSV infections or the diseases caused by such infections. Such method should be useful for limiting the severity of an RSV infection within an infected individual and the likelihood of transmission of the RSV infection from the infected individual to a non-infected individual.

Therefore, the present invention provides 3,3',4,4',5,5'-hexahydroxy-trans-stilbene or a pharmaceutically acceptable ester thereof for use in the treatment and prevention of infections by or of diseases caused by RSV. 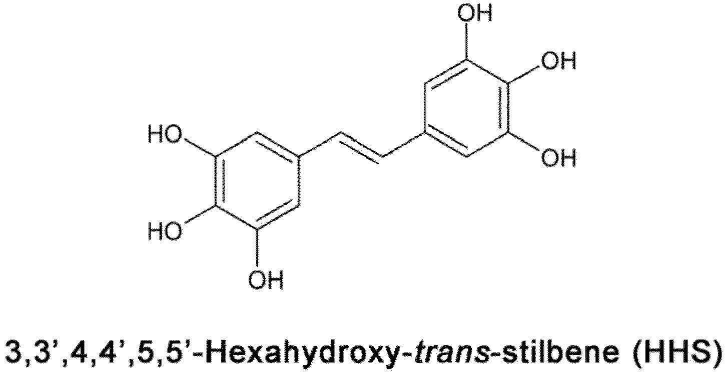 3,3',4,4',5,5'-hexahydroxy-trans-stilbene and pharmaceutically acceptable esters thereof according to the present invention was identified in the course of the present invention to exhibit realistic and effective inhibitory activity towards RSV. The compounds according to the present invention can therefore effectively prevent or hinder RSV from becoming pathogenic for human patients. Accordingly, the compounds according to the present invention are able to inhibit RSV from entering human cells at least to a certain extent to provide significant advantage for the patient to reduce the risk of developing diseases caused by these viruses. The compounds of the present invention are easily accessible, well tolerated and can successfully applied to humans without the need of invasive methods. Moreover, the compounds according to the present invention are regarded as "GRAS", i.e. they are "generally recognised as safe (i.a. under sections 201(s) and 409 of the US Federal Food, Drug, and Cosmetic Act.

Resveratrol, a stilbene compound (3, 5, 4'-trihydroxystilbene), has been reported to show promising antiviral activity against numerous viruses responsible for severe respiratory infections, such as influenza virus, respiratory syncytial virus and SARS-CoV-2, which are known to cause pneumonia, acute respiratory distress syndrome or multiorgan failure, especially, in vulnerable individuals like immunocompromised patients or the elderly (Filardo et al., Pharm Ther. 214 (2020), 107613; Liu et al., J. Virol. 88 (2014), 4229-4236). Resveratrol was assumed to have potential value for its anti-inflammatory activity, since most of the severe virus-associated complications are related to the over-activation of the host-immune response, leading to lung damage.

WO 00712534 A2 suggests the use of hydroxylated stilbenes against herpes viruses, including resveratrol and piceatannol (3,3',4,5'-tetrahydroxy-trans-stilbene), alleging both, the cis form or trans form being suitable for inhibiting the formation of herpes virus particles (see also: US 2023/089605 A1).

According to the present invention, 3,3',4,4',5,5'-hexahydroxy-trans-stilbene and pharmaceutically acceptable esters thereof have surprisingly turned out to be significantly more active in inhibiting RSV than comparable poly-phenolic substances suggested as being virus-inhibiting, such as resveratrol. Although resveratrol shows some inhibitory effect concerning RSV, 3,3',4,4',5,5'-hexahydroxy-trans-stilbene and pharmaceutically acceptable esters thereof have significantly enhanced inhibitory effect over resveratrol, as shown in detail in the example section of the present invention.

3,3',4,4',5,5'-hexahydroxy-trans-stilbene and pharmaceutically acceptable esters thereof used according to the present invention are preferably inhibiting replication of RSV, and/or for preventing the cytopathic effect of an active virus replication of RSV, and/or preventing viral infections RSV through airborne channels.

Stilbene derivatives according to the present invention were previously disclosed e.g. in WO 02/50007 A2, WO 02/057219 A1, WO 2005/016860 A1, WO 2007/002973 A2 and WO 2022/200086 A1.

These (poly-) hydroxylated phenols were described to have increased anti-cancer and anti-inflammatory effects in comparison to resveratrol due to the increased number of OH-groups in para position on polyhydroxylated stilbenes. Hexahydroxystilbene showed most effective anti-inflammatory and anti-cancer activity in vitro and anticancer effects in animal studies and was shown to inhibit HIV infection in vitro at a very early stage. These substances were also disclosed as regulators of T cells, neutrophils, macrophages and corresponding cytokines.

In CN 1736986 A, stilbene derivates were suggested as anti-viral substances for SARS-CoV-1 wherein the compounds disclosed to have antiviral activity were pyridine-group containing compounds and 2,2' -OH or CHsO- substituted compounds as well as 3, 3' methylbutenyl substituted compounds. Moreover, the nature of the compounds with actual anti-SARS activity were not disclosed.

3,3',4,4',5,5'-hexahydroxy-trans-stilbene and pharmaceutically acceptable esters thereof are excellent free radical scavengers, exhibiting a broad range of biochemical effects, such as inhibiting key enzymes of DNA synthesis or inflammation. In addition, some of them were shown to inhibit different virus infections through unspecific inhibition of virus entry or replication. Those compounds were selected due to their chemical structure and availability in natural sources such as tea or fruits.

In the course of the present invention, 3,3',4,4',5,5'-hexahydroxy-trans-stilbene and pharmaceutically acceptable esters thereof were investigated regarding their inhibitory effects on virus infection using in vitro cell culture models.

In contrast to other suggestions in the prior art in which several substances were described to have potential anti-RSV properties, it turned out with the present invention that polyphenolic substances highly vary in their effect against RSV and that only a few of these substances are effective.

These experiments performed in the course of the present invention showed that the synthetic resveratrol analogon according to the present invention and its pharmaceutically acceptable esters exhibit inhibiting activity against RSV which significantly outperforms other natural polyphenolic compounds, even the few natural polyphenolic compounds which also turned out to have a real-world antiviral activity against RSV (at least - for resveratrol - in human cells).

Preferred examples pharmaceutically acceptable esters of 3,3',4,4',5,5'-hexahydroxy-trans-stilbene for use according to the present invention include formyl ester, acetyl ester, propionyl ester, butyryl ester, fumaryl ester, sulfuryl ester, hydroxamate ester, and enanthate ester. Esterification of trans-stilbene compounds is well available to a person skilled in the art, e.g. by Tain et al. (Antiox. 10 (2021), 420), WO 2004/000302 A1, US 2017/0183290 A1, US 2014/134117 A1, and WO 2018/164662 A1. Specifically the pharmaceutically acceptable esters of 3,3',4,4',5,5'-hexahydroxy-trans-stilbene may be provided (e.g. as pro-drugs), for example as mono-, di-, tri-, tetra-, penta- or hexa-esters, preferably as mono-esters.

3,3',4,4',5,5'-hexahydroxy-trans-stilbene and pharmaceutically acceptable esters thereof used in the present invention are in principle known as pharmaceutical substances. Accordingly, the formulations already known and proven to be effective for delivery of these compounds to the human subject are also applicable for the present invention. The route of administration can be divided into enteral or parenteral, such as oral, sublingual, intramuscular, subcutaneous, nasal, oral mucosa, topical, especially a topical application to the eye, to the oral cavity, or to the lips; bronchial, pulmonary, skin, peritoneum or rectum, etc., preferably oral, sublingual or nasal, especially as an inhalable and/or aerosol composition or as a nasal or oral spray and/or as sublingual films or tablets and/or as lollipops. The compounds of the present invention or the pharmaceutical composition containing it can be administered in a unit dosage form. The dosage form for administration can be a liquid dosage form or a solid dosage form. For example, the liquid dosage form can be a true solution type, colloid type, microparticle dosage form, emulsion type, or suspension type. Examples of dosage forms include tablets; caplets; capsules, such as hard gelatin capsules and soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); sublingual films or tablets; lollipops; gels; liquid dosage forms suitable for oral or mucosal administration to a patient, including suspensions (e.g., aqueous or non-aqueous liquid suspensions, oil-in-water emulsions, or water-in-oil liquid emulsions), solutions, and elixirs. The compounds of the present invention can be made into ordinary preparations, and can also be slow-release agents, controlled-release agents, targeted preparations, and various particulate drug delivery systems, including inhalation systems (i.e. in the form of a kit comprising the pharmaceutical composition according to the present invention and an inhalator, such as the Diskhaler and other inhalators already used as inhalators for pharmaceutical compositions).

Preferably, the pharmaceutical composition is administered to an infected site during the prodromal stage of infection.

Therefore, the present invention also relates to the use of a pharmaceutical preparation comprising 3,3',4,4',5,5'-hexahydroxy-trans-stilbene and pharmaceutically acceptable esters thereof according to the present invention with a pharmaceutically acceptable excipient. A pharmaceutically acceptable excipient may be any excipient known to be suitable and used for the compounds according to the present invention, especially a carrier or diluent. According to a preferred embodiment, 3,3',4,4',5,5'-hexahydroxy-trans-stilbene is provided as the single active ingredient in the pharmaceutical preparation. This means that the pharmaceutical preparation contains 3,3',4,4',5,5'-hexahydroxy-trans-stilbene as the single effective agent with specific antiviral activity. Accordingly, no further antiviral agent in an effective antiviral amount is present in this preferred composition.

In order to make a unit dosage form, various carriers known in the art can be widely used.

Examples of carriers or diluents are, for example, absorbents, such as starch, dextrin, calcium sulfate, lactose, mannitol, sucrose, sodium chloride, glucose, urea, kaolin, microcrystalline cellulose, aluminium silicate, etc.; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone, etc.; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose, etc.; disintegration inhibitors, such as sucrose, glyceryl tristearate, cocoa butter, hydrogenated oil, etc.; absorption enhancers, such as quaternary ammonium salts, sodium lauryl sulfate, etc.; lubricants, such as talc, silicon dioxide, corn starch, stearic acid, boric acid, liquid paraffin, polyethylene glycol, etc.. Other carriers such as polyacrylic resins, liposomes, water-soluble carriers such as PEG4000 and PEG6000, PVP and so on. The tablets can also be further made into coated tablets, such as sugar-coated tablets, film-coated tablets, enteric-coated tablets, or double-layered and multi-layered tablets. For example, in order to make the administration unit into a pill, various carriers known in the art can be widely used. Examples of carriers are, for example, diluents and absorbents, such as glucose, lactose, starch, cocoa butter, hydrogenated vegetable oil, polyvinylpyrrolidone, kaolin, talc, etc.; binders, such as acacia, tragacanth, gelatin, Ethanol, honey, liquid sugar, rice paste or batter, etc.; disintegrants, such as agar powder, dried starch, alginate, sodium lauryl sulfonate, methyl cellulose, ethyl cellulose, etc.. For example, in order to make the dosing unit into a capsule, the compounds of the present invention as an active ingredient are mixed with the aforementioned various carriers, and the resulting mixture is placed in a hard gelatin capsule or a soft capsule. The active ingredient of the compound of the present invention can also be made into microcapsules, suspended in an aqueous medium to form a suspension, or filled into hard capsules or made into injections, inhalations or aerosols, for application. For example, the compounds of the present invention are prepared into an injection, an inhalation or aerosol preparation, such as a solution, a suspension solution, an emulsion, a lyophilized powder, and this preparation may be aqueous or non-aqueous.

The pharmaceutical preparation of the present invention may contain one and/or more pharmacodynamically acceptable excipients, such as carriers, diluents, binders, lubricants, preservatives, surfactants or dispersants. For example, the diluent can be selected from water, ethanol, polyethylene glycol, 1,3-propanediol, ethoxylated isostearyl alcohol, polyoxylated isostearyl alcohol, polyoxyethylene sorbitol fatty acid ester, etc.. In addition, in order to prepare an isotonic inhalation solution/suspension, aerosol, injection, an appropriate amount of sodium chloride, glucose or glycerin can be added to the injection preparation. In addition, conventional solubilizers, buffers, pH adjusters, etc. can also be added. These auxiliary materials are commonly used in this field. In addition, if necessary, colouring agents, preservatives, flavours, sweeteners, or other materials can also be added to the pharmaceutical preparation.

In order to achieve the purpose of medication and enhance the therapeutic effect, the drug or pharmaceutical composition of the present invention can be administered by any known administration method. The dosage of the compound or pharmaceutical composition of the present invention depends on many factors, such as the nature and severity of the disease to be prevented or treated, the gender, age, weight, personality and individual response of the patient or animal, the route of administration, the number of administrations and the purpose of treatment, so the therapeutic dose of the present invention can have a wide range of changes. Generally speaking, the dosage of the pharmacodynamic ingredient of the present invention is well known to those skilled in the art. According to the actual amount of the drug contained in the final formulation of the compound composition of the present invention, appropriate adjustments can be made to meet the requirements of the therapeutically effective dose, and the dosage of the compounds of the present invention can be completed, most preferably 0.1-20 mg/Kg body weight.

The above-mentioned dosage can be administered in a single dosage form or divided into several, for example, two, three or four dosage forms, which is limited by the clinical experience of the administering doctor and the dosage regimen including the use of other treatment means.

According to a preferred embodiment, 3,3',4,4',5,5'-hexahydroxy-trans-stilbene and pharmaceutically acceptable esters thereof according to the present invention are formulated for delivery into the upper respiratory system. Exemplary formulations include nasal, bronchial, oral, and pulmonary formulations. However, the compounds according to the present invention may also be formulated for topical administration including a liquid, gel, wax, or paste. It is specifically preferred to formulate the present composition as an aerosol. The aerosol can be a liquid or powdered aerosol. In some embodiments, the composition contains one or more pharmaceutically acceptable excipients such as glycerol. The composition can contain 0.01%-20% w/v of the effective ingredient according to the present invention and 10% to 20% glycerol.

Pharmaceutical compositions and unit dosage forms of the present invention typically also include one or more pharmaceutically acceptable excipients, especially carriers or diluents. Advantages provided by specific compounds of the disclosure, such as increased solubility and/or enhanced flow, purity, or stability (e.g., hygroscopicity) characteristics can make them better suited for pharmaceutical formulation and/or administration to patients than the prior art.

Suitable excipients are well known to those skilled in the art of pharmacy or pharmaceutics. Whether a particular excipient is suitable for incorporation into a pharmaceutical composition or dosage form depends on a variety of factors well known in the art including the way in which the dosage form will be administered to a patient. For example, oral dosage forms such as tablets or capsules may contain excipients not suited for use in parenteral dosage forms. The suitability of a particular excipient may also depend on the specific active ingredients in the dosage form. For example, the decomposition of some active ingredients can be accelerated by some excipients such as lactose, or when exposed to water. Active ingredients that include primary or secondary amines are particularly susceptible to such accelerated decomposition.

Specifically preferred pharmaceutically acceptable excipients are antioxidants (reduction agents). Antioxidants commonly used in pharmaceutical compositions include citric acid and its salts (E330-E333), tartaric acid and its salts (E334-E337), phosphoric acid and its salts (E338-E343) and ethylenediaminetetraacetic acid (EDTA) and its salts (calcium disodium EDTA, E385), vitamins C, E, etc..

The disclosure further encompasses pharmaceutical compositions and dosage forms that include one or more compounds that reduce the rate by which an active ingredient will decompose. Examples of such compounds are stabilizers, such as antioxidants such as ascorbic acid, pH buffers, or salt buffers. In addition, pharmaceutical compositions or dosage forms of the disclosure may contain one or more solubility modulators, such as sodium chloride, sodium sulfate, sodium or potassium phosphate or organic acids. A specific solubility modulator is tartaric acid.

Like the amounts and types of excipients, the amounts and specific type of compounds according to the present invention in a dosage form may depend on factors such as the route by which it is to be administered to patients. Typical dosage forms of the compounds of the present invention contain the effective ingredient according to the present invention in an amount of from about 100 µg to about 10 g, preferably in an amount of from about 1 mg to about 1 g, more preferably in an amount of from 10 mg to 500 mg, even more preferably in an amount of from about 20 mg to about 300 mg. Preferred dosage forms contain the compounds of the present invention in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

Preferably, the pharmaceutical compositions may also include a carrier, for example a sugar alcohol such as but not limited to glycerol, mannitol, sorbitol, xylitol, and erythritol. In a specific embodiment, the sugar alcohol is glycerol.

Typical topical dosage forms include liquids, creams, lotions, ointments, gels waxes, pastes, sprays, aerosols, solutions, emulsions, and other forms know to one of skill in the art. In a preferred embodiment, the present compounds are delivered to oral, nasal, or bronchial tissue in a suitable topical dosage form.

For non-sprayable topical dosage forms, viscous to semi-solid or solid forms including a carrier or one or more excipients compatible with topical application and having a dynamic viscosity preferably greater than water are typically employed. Suitable formulations include solutions, suspensions, emulsions, creams, ointments, powders, gels, waxes, pastes, liniments, salves, and the like, which are, if desired, sterilized or mixed with auxiliary agents (e.g., preservatives, stabilizers, wetting agents, buffers, or salts) for influencing various properties, such as, for example, osmotic pressure.

Nasal spray drug products contain therapeutically active ingredients dissolved or suspended in solutions or mixtures of excipients in non-pressurized dispensers that deliver a spray containing a metered dose of the active ingredient. The dose can be metered by the spray pump or could have been pre-metered during manufacture. A nasal spray unit can be designed for unit dosing or can discharge up to several hundred metered sprays of formulation containing the drug substance. Nasal sprays are applied to the nasal cavity for local and/or systemic effects.

According to another preferred embodiment, the compounds according to the present invention are provided as inhalation solution and suspension drug products. Such products are typically aqueous-based formulations that contain therapeutically active ingredients and can also contain additional excipients. Aqueous-based oral inhalation solutions and suspension must be sterile. Inhalation solutions and suspensions are intended for delivery to the lungs by oral inhalation for local and/or systemic effects and are to be used with a specified nebulizer. An inhalation spray drug product consists of the formulation and the container closure system. The formulations are typically aqueous based and must be sterile. Inhalation sprays are intended for delivery to the lungs by oral inhalation for local and/or systemic effects. Other suitable topical dosage forms include sprayable aerosol preparations wherein the active ingredient, preferably in combination with a solid or liquid inert carrier, is packaged in a mixture with a pressurized volatile (e.g., a gaseous propellant, such as freon), or in a squeeze bottle. Examples of sprayable aerosol preparations include metered dose inhalers, dry powder inhalers, and nebulizers. Moisturizers or humectants can also be added to pharmaceutical compositions and dosage forms if desired.

It may also be advantageous to provide the pharmaceutical compositions according to the present invention in transdermal and mucosal dosage forms, such as ophthalmic solutions, patches, sprays, aerosols, creams, lotions, suppositories, ointments, gels, solutions, emulsions, suspensions, or other forms known to one of skill in the art. Dosage forms suitable for treating mucosal tissues within the oral cavity can be formulated as mouthwashes, as oral gels, or as buccal patches. Additional transdermal dosage forms include reservoir type or matrix type patches, which can be applied to the skin and worn for a specific period of time to permit the penetration of a desired amount of active ingredient. Suitable excipients (e.g., carriers and diluents) and other materials that can be used to provide transdermal and mucosal dosage forms are well known to those skilled in the pharmaceutical field, and depend on the particular tissue or organ to which a given pharmaceutical composition or dosage form will be applied. With that fact in mind, typical excipients include water, acetone, ethanol, ethylene glycol, propylene glycol, butane-1,3-diol, isopropyl myristate, isopropyl palmitate, mineral oil, and mixtures thereof, to form dosage forms that are non-toxic and pharmaceutically acceptable. Depending on the specific tissue to be treated, additional components may be used prior to, in conjunction with, or subsequent to treatment with the compounds according to the present invention. For example, penetration enhancers can be used to assist in delivering the active ingredients to or across the tissue. Suitable penetration enhancers include acetone; various alcohols such as ethanol, oleyl, a tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as TWEEN 80 (polysorbate 80) and SPAN 60 (sorbitan monostearate).

The pH of a pharmaceutical composition or dosage form, or of the tissue to which the pharmaceutical composition or dosage form is applied, may also be adjusted to improve delivery of the active ingredient(s). Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of the active ingredient(s) so as to improve delivery. In this regard, stearates can serve as a lipid vehicle for the formulation, as an emulsifying agent or surfactant, and as a delivery-enhancing or penetration-enhancing agent.

The compounds of the present invention can also be formulated as extended or delayed release formulations. Extended and delayed release formulations for various active ingredients are known in the art, for example by encapsulation.

The compounds of the present invention are present in the pharmaceutical composition in about 0.001% to about 50% w/v, typically from about 0.01% to about 0.1% w/v, more typically about 1 % to about 20% w/v. In a preferred embodiment, compounds of the present invention are present in about 0.01% to about 20% w/v. The pharmaceutically acceptable excipient and eventually other compounds or agents present in the pharmaceutical composition then add up to the 100%.

The compounds and compositions of the present invention are useful for the treatment of one of more symptoms of viral infection with RSV. Preferably, the pharmaceutical compositions according to the present invention are formulated for nasal or oral application, such as drops, applicators, or sprays. One embodiment provides the compositions according to the present invention for prophylactically or therapeutically treating patients which are infected by or are at risk of being infected by RSV, especially for use to prevent viral infections through airborne channels.

According to a further aspect, the present invention relates to the use 3,3',4,4',5,5'-hexahydroxy-trans-stilbene or a pharmaceutically acceptable ester thereof, according to the present invention for the manufacture of a pharmaceutical preparation according to the present invention for the treatment and prevention of diseases, disorders and symptoms associated with or caused by RSV infections in a human subject, especially for inhibiting RSV. Preferably, the use is for inhibiting replication of RSV, and/or for preventing the cytopathic effect of an active virus replication of RSV, and/or preventing viral infections with RSV through airborne channels.

According to another aspect, the present invention relates to a method for the treatment and prevention of infections by or of diseases, disorders or symptoms caused by or associated with infectious RSV in a human subject, especially for inhibiting RSV, wherein to a subject which is infected by or are at risk of being infected by RSV, an effective amount of 3,3',4,4',5,5'-hexahydroxy-trans-stilbene or a pharmaceutically acceptable ester thereof according to the present invention or a pharmaceutical preparation according to the present invention is administered. This method is in particular suitable for inhibiting replication of RSV, and/or for preventing the cytopathic effect of an active virus replication of RSV, and/or preventing viral infections with RSV through airborne channels in a human subject.

The present invention is further illustrated by the following examples and the drawing figures, yet without being restricted thereto.

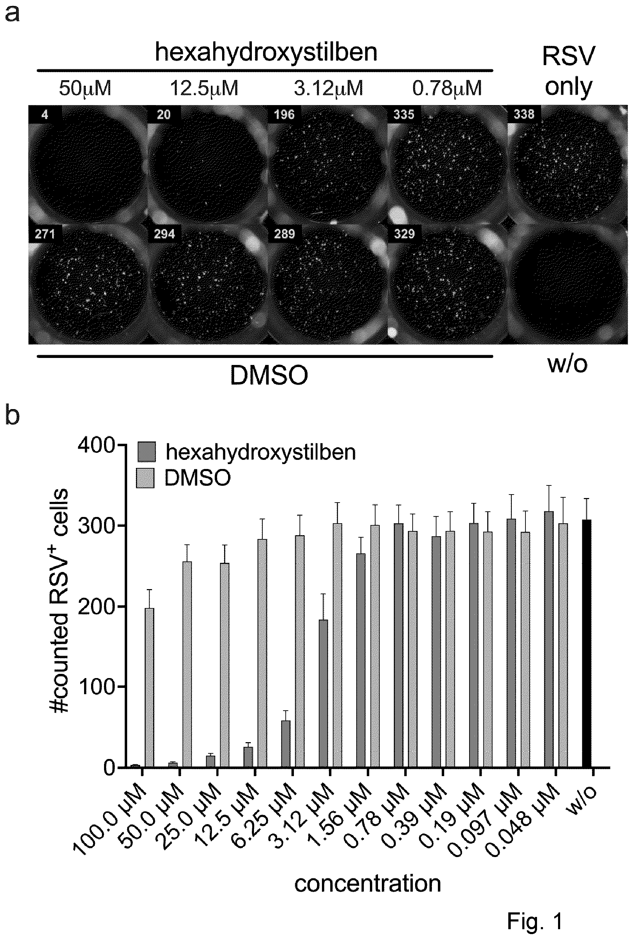

Fig.1 shows the *in vitro* antiviral activity of 3,3',4,4', 5, 5' - hexahydroxy-trans-stilbene against RSV. HEp2 cells were infected with RSV and concomitantly treated with 0.048-100 µM 3,3',4,4',5,5'-hexahydroxy-trans-stilbene. After 24h the RSV infected cells were stained with a RSV-specific recombinant monoclonal antibody followed by an Alexa Fluor^{™} Plus 488-labelled polyclonal goat anti-human IgG staining. The number of RSV infected cells were counted by an ImmunoSpot^{®} analyzer. (a) A representative result of RSV infected cells treated with different concentration of 3,3',4,4',5,5'-hexahydroxy-trans-stilbene or DMSO. As controls RSV infected cells without treatment (RSV only) or non-infected cells (w/o) are shown. (b) Summary of 5 independent RSV infection experiments in the absence (w/o) or presence of 3,3',4,4',5,5'-hexahydroxy-trans-stilbene (red bars) or DMSO (black bars). Data represent mean values±SEM.

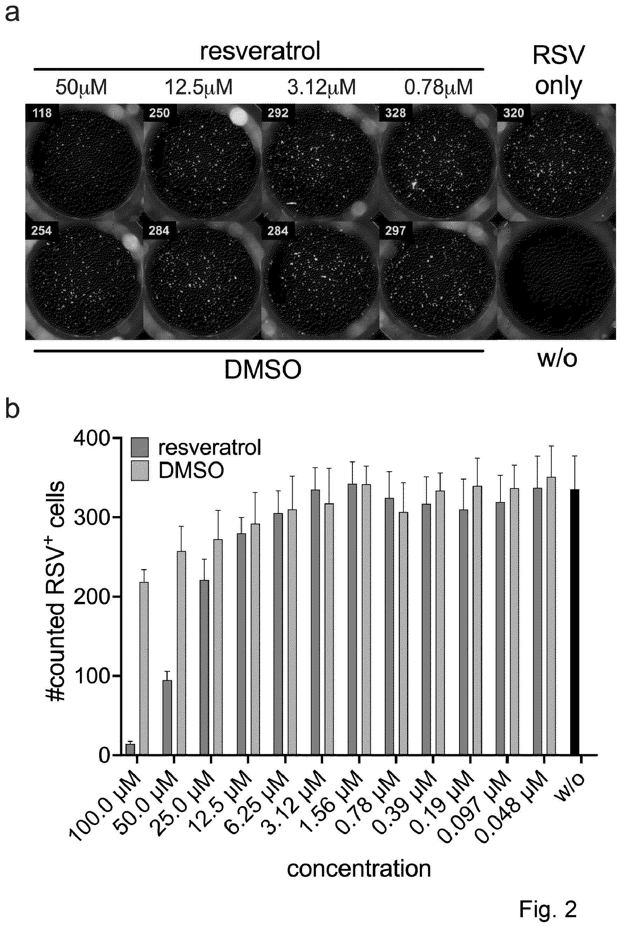

Fig.2 shows the *in vitro* antiviral activity of resveratrol against RSV. HEp2 cells were infected with RSV and concomitantly treated with 0.048-100 µM resveratrol. After 24h the RSV infected cells were stained with a RSV-specific recombinant monoclonal antibody followed by an Alexa Fluor^{™} Plus 488-labelled polyclonal goat anti-human IgG staining. The number of RSV infected cells were counted by an ImmunoSpot^{®} analyzer. (a) A representative result of RSV infected cells treated with different concentration of resveratrol or DMSO. As controls RSV infected cells without treatment (RSV only) or non-infected cells (w/o) are shown. (b) Summary of 3 independent RSV infection experiments in the absence (w/o) or presence of resveratrol (red bars) or DMSO (black bars). Data represent mean values with SEM.

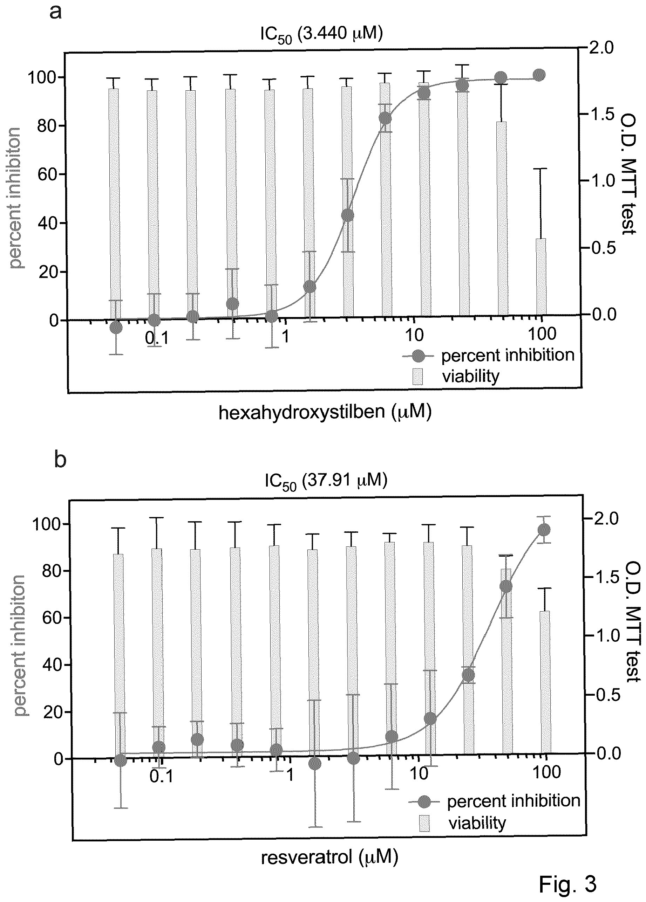

Fig. 3. shows the inhibition of RSV by 3,3',4,4',5,5'-hexahydroxy-trans-stilbene (a) or resveratrol (b). Percent inhibition of RSV infection in the presence of different concentrations of 3,3',4,4',5,5'-hexahydroxy-trans-stilbene and resveratrol calculated relative to the infection in virus only cells (red circles). IC₅₀ was calculated by nonlinear regression ((inhibitor) vs response - variable slope (four parameter)) using GraphPad Prism. Potential cytotoxic effect of 3,3',4,4',5,5'-hexahydroxy-trans-stilbene or resveratrol on the HEp2 cells was assessed by the MTT assay (black triangles). Graphs show the mean with 95% CI from 5 and 3 independent experiments for 3,3',4,4',5,5'-hexahydroxy-trans-stilbene and resveratrol, respectively.

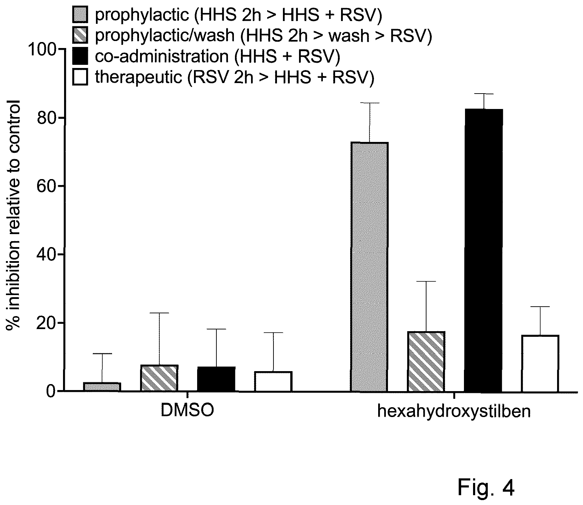

Fig. 4. shows the anti-viral effect of 3,3',4,4',5,5'-hexahydroxystilben applied at different timepoint relative to viral infection. Prophylactic indicates 8 µM of 3,3',4,4',5,5'-hexahydroxystilben treatment 2 hours prior infection (grey bars), prophylactic/wash represents pre-administration for 2 hours followed by wash prior infection (grey bars with stripes), co-administration represents treatment at the time of infection (black bars) and therapeutic administration indicates treatments 2 hours post-infection (white bars). Graphs show the mean with 95% CI from 4 independent experiments.

### Examples:

In the present examples, human RSV inhibiting effects of 3,3',4,4',5,5'-hexahydroxy-trans-stilbene were investigated in comparison with resveratrol.

### Materials and Methods

### Cell assay

RSV-permissive HEp2 cells (1.75×10⁴ cells/per well in 100µl DMEM supplemented with 2mM L-glutamine/10% FCS) were seeded in 96-well plates. After reaching 80-90% confluence, cells were infected with RSV at a predetermined dilution resulting in approximately 300 infected cells when stained 24 hours post-infection. HEp2 cells were infected with RSV in the absence or presence of different concentrations of hexahydroxystilbene (3,3',4,4',5,5'-hexahydroxy-trans-stilbene; HHS), resveratrol (RES) or a buffer control. The concentrations of HHS and RES were between 100 µM and 0.048 µM. After 24 hours of incubation, RSV-infected HEp2 cells were treated with a humanised RSV-specific antibody (palivizumab). After one hour incubation, the second fluorescent anti-human IgG antibody was added. The labelled virus-infected cells were counted with an ImmunoSpot^{®} analyser (C.T.L.). Percent inhibition of RSV infection was calculated relative to 100% infection counted in HEp2 cells with RSV only and the IC₅₀ values were determined with nonlinear regression ((inhibitor) vs response - variable slope (four parameter)) using GraphPad Prism 9.5.1 (GraphPad Software, Inc., La Jolla, CA, USA). In addition, an MTT killing assay was performed to assess the viability of cells treated with HHS or RES.

### Substances

3,3',4,4',5,5'-hexahydroxy-trans-stilbene and resveratrol were dissolved in DMSO (Sigma) to substance stock concentrations of 10 mM. Substance stocks were prepared freshly prior to every assay. Further dilutions were made in DMEM supplemented with 2mM L-glutamine/10% FCS to gain the final assay concentrations.

### Results

To investigate whether 3,3',4,4',5,5'-hexahydroxy-trans-stilbene and resveratrol inhibit the replication of RSV, assays in RSV-infected HEp2 cells were performed. In these experiments, 3,3',4,4',5,5'-hexahydroxy-trans-stilbene and resveratrol showed a significant inhibition of RSV replication. The inhibition capacity of 3,3',4,4',5,5'-hexahydroxy-trans-stilbene (IC₅₀ of 3.440 µM; Figs. 1 and 3a) was, however, about a factor of 10 higher than the inhibition capacity of resveratrol (IC₅₀ of 37.91 µM; Figs. 2 and 3b). Importantly, no cytotoxicity was observed in the MTT assay with HEp2 with 3,3',4,4',5,5'-hexahydroxy-trans-stilbene used at a concentration of 25 µM or less (Fig. 3).

To investigate the mode of action of 3,3',4,4',5,5'-hexahydroxy-trans-stilbene on RSV inhibition, we repeated infection experiments using 8 µM of 3,3',4,4',5,5'-hexahydroxy-trans-stilbene. In addition to the previous experimental settings in which RSV and 3,3',4,4',5,5'-hexahydroxy-trans-stilbene were applied simultaneously to HEp2 cells (Fig. 4, co-administration), we performed prophylactic and therapeutic settings by applying 3,3',4,4',5,5'-hexahydroxy-trans-stilbene before or after RSV infections. In the prophylactic setting, HEp2 cells treated with 3,3',4,4',5,5'-hexahydroxy-trans-stilbene 2 hours prior infection and during infection showed similar infection levels when compared to the co-administration (Fig. 4, prophylactic). However, in the prophylactic setting if 3,3',4,4',5,5'-hexahydroxy-trans-stilbene was removed prior to infection, the inhibition of RSV infection was greatly reduced (Fig. 4, prophylactic/wash). Similarly, the therapeutic setting, where 3,3',4,4',5,5'-hexahydroxy-trans-stilbene was applied 2 hours after infection, resulted in reduced inhibition of RSV infection (Fig. 4, therapeutic). Taken together, this shows that the presence of 3,3',4,4',5,5'-hexahydroxy-trans-stilbene during RSV infection is required for optimal inhibition.

In view of this disclosure and the examples, the following preferred embodiments are disclosed with the present specification:
1. 3,3',4,4',5,5'-hexahydroxy-trans-stilbene or a pharmaceutically acceptable ester hereof for use in the treatment and prevention of infections by or of diseases, disorders or symptoms caused by or associated with infectious human respiratory syncytial virus (RSV).
2. 3,3',4,4',5,5'-hexahydroxy-trans-stilbene or a pharmaceutically acceptable ester thereof for inhibiting formation of RSV particles in a host cell.
3. 3,3',4,4',5,5'-hexahydroxy-trans-stilbene or a pharmaceutically acceptable ester thereof for blocking replication of an RSV in a cell infected with RSV.
4. 3,3',4,4',5,5'-hexahydroxy-trans-stilbene or a pharmaceutically acceptable ester thereof for use according to any one of embodiments 1 to 3, wherein 3,3',4,4',5,5'-hexahydroxy-trans-stilbene or a pharmaceutically acceptable ester thereof is administered prior to or within 6 hours of infection of the host cell with RSV.
5. 3,3',4,4',5,5'-hexahydroxy-trans-stilbene or a pharmaceutically acceptable ester thereof for use according to any one of embodiments 1 to 4, wherein the pharmaceutically acceptable ester thereof is selected from formyl ester, acetyl ester, propionyl ester, butyryl ester, fumaryl ester, sulfuryl ester, hydroxamate ester, and enanthate ester, preferably a mono-, di-, tri-, tetra-, penta- or hexa-ester of any one of these esters, especially a mono-ester thereof.
6. 3,3',4,4',5,5'-hexahydroxy-trans-stilbene or a pharmaceutically acceptable ester thereof for use according to any one of embodiments 1 to 5, wherein 3,3',4,4',5,5'-hexahydroxy-trans-stilbene or a pharmaceutically acceptable ester thereof is included in a pharmaceutical composition which further comprises a topical carrier and which is administered is by topical administration to the skin.
7. 3,3',4,4',5,5'-hexahydroxy-trans-stilbene or a pharmaceutically acceptable ester thereof for use according to any one of embodiments 1 to 6, wherein inhibiting RSV is inhibiting replication of RSV and/or for preventing the cytopathic effect of an active virus replication of RSV and/or preventing viral infections with RSV through airborne channels.
8. A pharmaceutical preparation comprising or consisting of 3,3',4,4',5,5'-hexahydroxy-trans-stilbene or a pharmaceutically acceptable ester thereof according to any one of embodiments 1 to 7 as active ingredient, preferably as the single active ingredient, and a pharmaceutically acceptable excipient for use in the treatment and prevention of infections by or of diseases caused by infectious RSV.
9. A pharmaceutical preparation for use according to embodiment 8, wherein the preparation is for oral, sublingual, intramuscular, subcutaneous, nasal, oral mucosa, topical, especially a topical application to the eye, to the oral cavity, or to the lips; bronchial, pulmonary, skin, peritoneum or rectum administration, preferably for oral, sub-lingual or nasal administration, especially wherein the preparation is provided as an inhalable and/or aerosol composition or as a nasal or oral spray and/or as sublingual films or tablets and/or as lollipops.
10. A pharmaceutical preparation for use according to embodiment 8 or 9, wherein the pharmaceutical composition is administered to an infected site during the prodromal stage of infection.
11. A pharmaceutical preparation for use according to any one of embodiments 8 to 10, wherein the pharmaceutical is applied at or proximate a known site of infection or a site which is suspected of being infected.
12. A pharmaceutical preparation for use according to any one of embodiments 8 to 11, for reducing the symptoms of an RSV infection.
13. A pharmaceutical preparation for use according to any one of embodiment 8 to 12, wherein the pharmaceutically acceptable excipient is selected from diluents, such as water, acetone, ethanol, polyethylene glycol, polypropylene glycol, 1,3-propanediol, butane-1,3-diol, isopropyl myristate, isopropyl palmitate, mineral oil, ethoxylated isostearyl alcohol, polyoxylated isostearyl alcohol, polyoxyethylene sorbitol fatty acid ester; carrier, such as glycerol, mannitol, sorbitol, xylitol, and erythritol absorbents, such as starch, dextrin, calcium sulfate, lactose, mannitol, sucrose, sodium chloride, glucose, urea, kaolin, microcrystalline cellulose, aluminium silicate; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose; disintegration inhibitors, such as sucrose, glyceryl tristearate, cocoa butter, hydrogenated oil; absorption enhancers, such as quaternary ammonium salts, sodium lauryl sulfate; lubricants, such as talc, silicon dioxide, corn starch, stearic acid, boric acid, liquid paraffin, polyethylene glycol; polyacrylic resins, liposomes, water-soluble carriers such as PEG4000 and PEG6000, PVP; antioxidants; pH buffers and/or salt buffers; solubility modulators; penetration enhancers; antioxidants, such as citric acid and its salts, tartaric acid and its salts, phosphoric acid and its salts, and ethylenediaminetetraacetic acid (EDTA) and its salts, vitamins C, E; or mixtures thereof.
14. A pharmaceutical preparation for use according to any one of embodiments 8 to 13, wherein the preparation is an inhalation or aerosol preparation, especially an isotonic inhalation solution or suspension or a liquid or powdered aerosol.
15. A pharmaceutical preparation for use according to any one of embodiments 8 to 14, wherein the preparation contains 3,3',4,4',5,5'-hexahydroxy-trans-stilbene or a pharmaceutically acceptable ester thereof in an amount of from about 100 µg to about 10 g, preferably in an amount of from about 1 mg to about 1 g, more preferably in an amount of from 10 mg to 500 mg, even more preferably in an amount of from about 20 mg to about 300 mg.
16. A pharmaceutical preparation for use according to any one of embodiments 8 to 15, wherein the preparation contains 3,3',4,4',5,5'-hexahydroxy-trans-stilbene or a pharmaceutically acceptable ester thereof in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.
17. A pharmaceutical preparation for use according to any one of embodiments 8 to 16, wherein the preparation is contained in a metered dose inhaler, in a dry powder inhaler, or in a nebulizer.
18. A pharmaceutical preparation for use according to any one of embodiments 8 to 17, wherein the preparation contains 3,3',4,4',5,5'-hexahydroxy-trans-stilbene or a pharmaceutically acceptable ester thereof in 0.001% to 50% w/v, preferably in 0.01% to 20% w/v, more preferred in 0.1% to 20% w/v, especially in 1 % to 20% w/v.
19. Use of 3,3',4,4',5,5'-hexahydroxy-trans-stilbene or a pharmaceutically acceptable ester thereof according to any one of embodiments 1 to 7 for the manufacture of a pharmaceutical preparation according to any one of embodiments 8 to 18 for the treatment and prevention of diseases caused by RSV.
20. Use according to embodiment 19 for inhibiting replication of a RSV, and/or for preventing the cytopathic effect of an active virus replication of RSV and/or preventing viral infections with RSV through airborne channels.
21. Method for the treatment and prevention of diseases caused by RSV, in a human subject, especially for inhibiting an RSV, wherein to a subject which is infected by or are at risk of being infected by RSV, an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18 is administered.
22. Method for inhibiting replication of RSV, and/or for preventing the cytopathic effect of an active virus replication of RSV, and/or preventing viral infections with RSV through airborne channels in a human subject, wherein to a subject which is infected by or are at risk of being infected by RSV, an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18 is administered.
23. A kit comprising
   (1) a container comprising an effective amount of 3,3',4,4',5,5'-hexahydroxy-trans-stilbene or a pharmaceutically acceptable ester thereof according to any one of embodiments 1 to 7 or a pharmaceutical preparation according to any one of embodiments 8 to 18, suitable for inhalation; and
   (2) an inhalator.

## Claims

1. 3,3',4,4',5,5'-hexahydroxy-trans-stilbene or a pharmaceutically acceptable ester thereof, for use in the treatment and prevention of infections with human respiratory syncytial virus (RSV) in a human subject.

2. 3,3',4,4',5,5'-hexahydroxy-trans-stilbene or a pharmaceutically acceptable ester thereof, for use according to claim 1, wherein inhibiting RSV is inhibiting replication of RSV and/or for preventing the cytopathic effect of an active virus replication of RSV and/or preventing viral infections with RSV through airborne channels.

3. A pharmaceutical preparation comprising or consisting of 3,3',4,4',5,5'-hexahydroxy-trans-stilbene or a pharmaceutically acceptable ester thereof, for use according to claim 1 or 2 as active ingredient, preferably as the single active ingredient, and a pharmaceutically acceptable excipient.

4. A pharmaceutical preparation for use according to claim 3, wherein the preparation is for oral, sublingual, intramuscular, subcutaneous, nasal, oral mucosa, bronchial, pulmonary, skin, peritoneum or rectum administration, preferably for oral, sub-lingual or nasal administration.

5. A pharmaceutical preparation for use according to claim 3 or 4, wherein the preparation is provided as an inhalable and/or aerosol composition or as a nasal or oral spray and/or as sublingual films or tablets and/or as lollipops.

6. A pharmaceutical preparation for use according to any one of claims 3 to 5, wherein the pharmaceutically acceptable excipient is selected from diluents, such as water, acetone, ethanol, polyethylene glycol, polypropylene glycol, 1,3-propanediol, butane-1,3-diol, isopropyl myristate, isopropyl palmitate, mineral oil, ethoxylated isostearyl alcohol, polyoxylated isostearyl alcohol, polyoxyethylene sorbitol fatty acid ester; carrier, such as glycerol, mannitol, sorbitol, xylitol, and erythritol absorbents, such as starch, dextrin, calcium sulfate, lactose, mannitol, sucrose, sodium chloride, glucose, urea, kaolin, microcrystalline cellulose, aluminium silicate; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose; disintegration inhibitors, such as sucrose, glyceryl tristearate, cocoa butter, hydrogenated oil; absorption enhancers, such as quaternary ammonium salts, sodium lauryl sulfate; lubricants, such as talc, silicon dioxide, corn starch, stearic acid, boric acid, liquid paraffin, polyethylene glycol; polyacrylic resins, liposomes, water-soluble carriers such as PEG4000 and PEG6000, PVP; antioxidants; pH buffers and/or salt buffers; solubility modulators; penetration enhancers; antioxidants, such as citric acid and its salts, tartaric acid and its salts, phosphoric acid and its salts, and ethylenediaminetetraacetic acid (EDTA) and its salts, vitamins C, E; or mixtures thereof.

7. A pharmaceutical preparation for use according to any one of claims 3 to 6, wherein the preparation is an inhalation or aerosol preparation, especially an isotonic inhalation solution or suspension or a liquid or powdered aerosol.

8. A pharmaceutical preparation for use according to any one of claims 3 to 7, wherein the preparation contains 3,3',4,4',5,5'-hexahydroxy-trans-stilbene or a pharmaceutically acceptable ester in an amount of from about 100 µg to about 10 g, preferably in an amount of from about 1 mg to about 1 g, more preferably in an amount of from 10 mg to 500 mg, even more preferably in an amount of from about 20 mg to about 300 mg.

9. A pharmaceutical preparation for use according to any one of claims 3 to 8, wherein the preparation contains 3,3',4,4',5,5'-hexahydroxy-trans-stilbene or a pharmaceutically acceptable ester in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

10. A pharmaceutical preparation for use according to any one of claims 3 to 9, wherein the preparation is contained in a metered dose inhaler, in a dry powder inhaler, or in a nebulizer.

11. A pharmaceutical preparation for use according to any one of claims 3 to 9, wherein the preparation contains 3,3',4,4',5,5'-hexahydroxy-trans-stilbene or a pharmaceutically acceptable ester in 0.001% to 50% w/v, preferably in 0.01% to 20% w/v, more preferred in 0.1% to 20% w/v, especially in 1 % to 20% w/v.

12. Use of 3,3',4,4',5,5'-hexahydroxy-trans-stilbene or a pharmaceutically acceptable ester thereof according to claim 1 or 2 for the manufacture of a pharmaceutical preparation according to any one of claims 3 to 11 for the treatment and prevention of a RSV infection or a disease caused by a RSV infection a human subject.

13. Use according to claim 12 for inhibiting replication of human RSV and/or for preventing the cytopathic effect of an active virus replication of human RSV and/or preventing viral infections with human RSV through airborne channels.

14. Method for the treatment and prevention of human RSV in a human subject, especially for inhibiting human RSV, wherein to a subject which is infected by or are at risk of being infected by human RSV an effective amount of 3,3',4,4',5,5'-hexahydroxy-trans-stilbene or a pharmaceutically acceptable ester thereof according to claim 1 or 2 or a pharmaceutical preparation according to any one of claims 3 to 11 is administered.

15. Method for inhibiting replication of RSV in a human subject, especially for inhibiting human RSV and/or for preventing the cytopathic effect of an active virus replication of RSV and/or preventing viral infections with RSV through airborne channels in a human subject, wherein to a subject which is infected by or are at risk of being infected by RSV an effective amount of 3,3',4,4',5,5'-hexahydroxy-trans-stilbene or a pharmaceutically acceptable ester thereof according to claim 1 or 2 or a pharmaceutical preparation according to any one of claims 3 to 11 is administered.
